# EUROPEAN PATENT SPECIFICATION

(11) **EP 1 523 967 B1**
(45) Date of publication and mention of the grant of the patent: **23.05.2007**
(21) Application number: 04024344.6
(22) Date of filing: 13.10.2004
(51) Int. Cl.: A61F 5/443

(54) **Ostomy pouch attachment adhesives resistant to stomal effluent**
Klebemittel zum Befestigen eines Ostomie-Beutels mit Beständigkeit gegen Stomaausfluss
Adhésifs pour attacher une poche d'ostomie présentant une résistance à l'effluent stomal

(30) Priority: 14.10.2003 US 510993 P
(43) Date of publication of application: 20.04.2005
(73) Proprietor: Bristol-Myers Squibb Company, New York, N.Y. 10154 (US)
(72) Inventor: Fattman, George F., Mt. Laurel, New Jersey 08054 (US); Sambasivam, Mahesh, Pennington, New Jersey 08534 (US)
(74) Representative: Holmes, Miles Keeton

(56) References cited:
- EP-A- 0 672 399
- EP-A- 1 424 088
- WO-A-96/03167
- WO-A-99/26565
- US-A- 5 015 244
- US-A1- 2003 004 477

## Description

This application claims the benefit of U.S. Provisional Application No. 60/510,993, filed 14 October, 2003 and is hereby incorporated by reference in its entirety.

### FIELD OF THE INVENTION

This invention relates to an adhesive for use in an ostomy device, in particular, an adhesive that is resistant to stomal effluent.

### BACKGROUND OF THE INVENTION

The most common commercial ostomy devices have two basic requirements for acceptable performance, attachment of the device to the peristomal area and containment of effluent from the stoma. These requirements are met by two discreet components of the device, referred to as the base plate or wafer (for attachment to the body) and the pouch or bag (for collection or containment of effluent). The wafer and pouch work together to protect the peristomal skin and provide a secure means for discrete collection and disposal of effluent.

The wafer and pouch components may be coupled together by a variety of means including thermal, ultrasonic, radio frequency, or other kinds of plastic welds, hot melt or reactive or curable glues, pressure sensitive adhesives, and various mechanical means. When these components cannot be easily separated or cannot be separated without causing damage to the device, for example when the coupling mechanism is a plastic weld or glue joint, then the product is a one piece appliance. Two piece appliances are devices in which the pouch and wafer components can be separated while preserving the usefulness of one or both components. Two piece devices can be made using coupling mechanisms based on pressure sensitive adhesives or from mechanical couplings of various designs. One design of a commercially successful mechanical coupling is described by British Patent specification GB 1 571 657.

Both one piece and two piece ostomy devices have advantages and disadvantages. The one piece design is very flexible, conforms well to the body, and is less noticeable under clothing compared with two piece products which tend to be more rigid and higher profile. In a two piece design, the wafer and pouch can be separated, and preferably separated and connected repeatedly using the same wafer and pouch, without compromising the effectiveness of at least one of the device components. The two piece design offers several advantages, the primary one being that the pouch can be removed and changed without having to change the wafer, which requires additional work and stresses the skin. Other advantages of the two piece design are that the stoma and peristomal area can be observed and, if needed, treated without removing the wafer or destroying the pouch. One piece products generally do not provide these advantages.

In the preceding review it can be seen that the coupling mechanism is of significant importance in determining the usefulness of an ostomy device since it strongly influences the flexibility, convenience, and versatility of the device. It also impacts the most important need for the wearer, containment of effluent. Like the other components of the device, the coupling must contain all effluent from the stoma, which may be emitted in solid, liquid or gaseous forms. Permanent couplings like thermal welds between the wafer and pouch are simple and effective mechanisms for reliable containment of stoma output but suffer the drawbacks of the one piece design. For this reason mechanical couplings that yield two piece products are sometimes preferred. However, mechanical couplings are more rigid and have a higher profile compared with the couplings of one piece designs, and so they can be uncomfortable and less discrete for the wearer. They also require sufficient manual dexterity and visual acuity to properly assemble or disassemble. However, a properly designed mechanical coupling like the one described in GB 1 571 657 yields a pouch to wafer connection that provides excellent security and containment characteristics.

Coupling mechanisms based on bonding together an ostomy pouch and wafer using pressure sensitive adhesives can combine the advantages of both one piece and two piece designs if they enable repeatable and secure attachment of the pouch and wafer, yield a low profile device, and provide reliable containment of effluent. A pressure sensitive adhesive based ostomy device coupling is defined herein as comprising a pressure sensitive adhesive, in adhesive contact with the surface of at least one other ostomy device component.

There are numerous prior art disclosures that describe the use of adhesives in ostomy device couplings. UK Patent 1,274,382 discloses a post surgical drainage device for receiving discharge from a surgical wound or stoma, and an attachment means for securing that device to the patient's body. The attachment means is comprised of two laminar parts assembled in face to face relationship. The first part of the attachment means comprises an adhesive for securing the attachment means to the body. The second part of the attachment means is a flexible plastic sheet that secures to the pouch by means of an adhesive.

US Patent 4,701,169 discloses an ostomy appliance comprising a body-attachable pad and a means whereby a plurality of ostomy bags can be adhesively attached to the pad in sequence by exposing a fresh layer of adhesive each time it is desired to attach a clean bag.

US Patent 5,160,330 discloses a water closet disposable pouch having an adhesive disc secured to an inside wall of the bag for use in connecting the bag to a flange worn on the body.

US Patent 5,496,296 discloses an adhesively coupled ostomy pouch and faceplate device wherein the faceplate contains an adhesive that may be squeezed out from a compartment in the faceplate to form a peristomal gasket.

US Patent 5,709,673 discloses a flushable ostomy pouch of either a one piece or two piece design wherein the two piece design includes an adhesive coupling to join together the wafer and pouch.

US Patent 5,722,965 discloses an adhesively coupled ostomy device comprising a repositionable foam tape that inhibits the formation of wrinkles in the coupling mechanism.

Patent Application WO99/26565 discloses an ostomy system comprising a pouch that is adhesively coupled to a mounting wafer. The mounting wafer has a landing zone film of releaseable plastic. The landing zone has one portion that is immovable with respect to the body surface and another part that is deflectable away from the body surface to which it is attached.

Patent application WO00/30576 discloses an ostomy appliance comprising a body side member and a receiving bag each including a flange said to be designed for removable adhesive connection of the appliance components wherein the collecting bag flange incorporates perforations to reduce the risk of leakage.

Patent application WO01/85074 discloses a carrier device comprising a base plate with flange for adhesive connection with an ostomy collecting bag wherein the outer portion of the flange is free to move relative to the base plate.

The preceding patents and patent applications all disclose ostomy device couplings in which both the body attaching and effluent collecting components have essentially only planar regions whereby they are attached together using an adhesive. There are additional prior art disclosures that describe ostomy device couplings made from flexible components that are substantially non-planar and which function by being assembled in more than two dimensions. US Patent 4,808,173 discloses a coupling ring assembly for an ostomy device in which one of the rings has a radially-facing annular channel for receiving a latching flange of another ring. A deformable, viscoelastic polymeric material lines the channel where the two coupling rings are attached. It is stated that additional security may be obtained if the liner also has adhesive properties.

US Patent 4,826,495 discloses a water closet flushable ostomy bag and a retainer plate to which it may be coupled by means of a pressure sensitive adhesive. The retainer plate includes a hollow bead projecting into the pouch which is said to provide a passage way for flatus to pass out of the pouch.

US Patent 5,429,626 discloses an ostomy appliance having a mounting member for attachment to the skin, the mounting member comprising a rigid mounting plate on its distal side and an axially extending curb for alignment of the pouch. The pouch is provided with an annular band of adhesive to removably adhere it to the mounting plate.

US Patent 5,346,482 discloses a two piece ostomy appliance comprising a flushable pouch that may be attached to an ostomy faceplate with an adhesive. The faceplate component has an axially extending protective collar that inserts into the pouch to shield water-soluble layers of the pouch from the fluids that enter it.

Patent Application WO97/35534 discloses a coupling device for mounting an ostomy bag to the body comprising annular sealing elements and annular bonding regions on both the pouch and wafer. At least one of the bonding regions is provided with a layer of repositionable adhesive. The sealing elements cooperate to form a water tight seal while the bonding regions to form a releaseable bond.

US Patent 5,800,415 discloses an ostomy collecting system comprised of a collecting bag and a base plate, each of which has an annular flange for adhesive coupling. Also disclosed is an axially extending collar that acts as a guide surface to prevent improper mounting of the bag and which forms a passage for the stoma, providing protection of the coupling adhesive from stomal effluent.

The objects of the invention are pressure sensitive adhesive compositions that have improved resistance to intestinal fluid and devices comprising these adhesives for collection of intestinal fluid or effluent from a stoma. These adhesive compositions may be used to couple a fluid or effluent collecting device component to a body attaching device component. They may also be used to attach any components of the device that may be exposed to stomal effluent.

It is generally known that intestinal fluids can vary greatly in their ability to attack the component materials of ostomy devices. It is believed that the variation in potency of intestinal fluids derives from many factors that influence the strength of their surface and enzymatic activities. These factors vary from individual to individual according to physiological factors and diet, among other causes. Another factor affecting the performance of ostomy devices is the wearing time during which the device is in contact with effluent. As a result, the same component designs and materials may be satisfactory for some individuals and unacceptable for others. Accordingly, the ability of a coupling device to provide sufficient containment of stoma effluent also varies according to its design and composition.

When a containment failure occurs in an ostomy device coupled by either plastic welds or by a mechanical means it is usually an unforeseen catastrophic failure that happens without warning. In contrast it has been found that ostomy devices coupled by pressure sensitive adhesives tend to lose containment over a longer period of time. Leaking may not occur all at once but can happen gradually by a migration of the intestinal fluid across the adhesively bonded interface. Those skilled in the art recognize that the strength of an adhesive bond is usually measured by peeling it from its adherend. Bond strength is a strong function of the application pressure used to create the bond and the amount of time that pressure is maintained. For pressure sensitive adhesive couplings used to connect an ostomy device component, it would be expected that the adhesives with higher bond strength would better contain the contents of an ostomy device. However, it has surprisingly been found that the chemical composition of the adhesive is a more significant factor.

It is believed that no prior art exists wherein the composition of a resealable adhesive coupling resistant to intestinal fluids is described as in the present invention. Of the 15 adhesive coupling patents or applications reviewed above, five are silent as to the composition of the adhesive. Eight suggest the general suitability of acrylic adhesives for ostomy couplings, particularly the grades of these adhesives indicated for contact with the skin. The remaining two suggest the use of hot melt adhesives, although the design of the couplings in these patents includes axially extended components of the coupling, which alleviates stress on the adhesive. Not only does the prior art not disclose the present invention of adhesives suitable for ostomy device couplings that are resistant to intestinal fluid, but also it is believed that some of the coupling designs disclosed are intended to compensate for the adhesive's inability to resist intestinal fluid. These axially extended designs also have a higher profile for the wearer and suffer the disadvantages of being higher profile, more visible to others, less flexible to the wearer, or all of the above.

Examples of the prior art include US Patent 5,496,296, which suggests the use of a conventional medical grade acrylic adhesive. Another example of an adhesive coupling based on acrylic polymers is disclosed in US Patent 5,722,965 which describes a resealable foam tape with a hypoallergenic acrylic adhesive. This adhesive has been found to yield an ostomy device as described in patent application WO99/26565 that successfully contains stomal effluent for several days. However, during that time effluent can be observed to migrate slowly across the adhesively coupled interface, again depending on physiological factors. As the migrating front approaches the radial edge of the coupling there is less and less adhesive in contact with the mating surfaces of the coupling, the coupling is less and less secure, and a leak is more and more imminent. An actual leak or the threat of an impending leak is incompatible with the satisfactory performance of the product. For longer wearing times or for individuals with more aggressive effluent an improvement would be obtained if the coupling retarded or eliminated stool migration and / or the leakage of effluent within the coupling interface.

### DESCRIPTION OF THE INVENTION

The present invention is defined in the claims.

The preferred embodiments of the invention defined by the claims illustrate an ostomy device that comprises an attachment component for attachment to the body, an effluent containment component, and a coupling mechanism to couple the two components together. The coupling mechanism includes an adhering component with an adhesive that comprises either a multi-block copolymer of vinyl aromatic and olefin comonomers, or poly(ethylene vinyl acetate), or combinations thereof. The multi-block copolymer of a vinyl aromatic and olefin may be selected from the group consisting of poly(styrene-isoprene-styrene), poly(styrene-butadiene-styrene), poly(styrene ethylene-butylene-styrene), poly (styrene-ethylene-propylene-styrene), poly (styrene-isobutylene-styrene), poly(styrene-ethylene-ethylene-propylene-styrene), and combinations thereof. The multi-block copolymer of vinyl aromatic and olefin comonomers comprises from about 20 to about 85 percent by weight of the dry adhesive, preferably from about 30 to about 60% by weight of the dry adhesive. The multiblock copolymer of vinyl aromatic and olefin is blended with a plasticizer, wherein the plasticizer comprises from 0 to about 40 percent by weight of the dry adhesive. In addition, the multi-block copolymer of vinyl aromatic and olefin comonomers is blended with a tackifier, wherein the tackifier comprises from about 5 to about 60 percent of the dry adhesive.

The multi-block copolymer of vinyl aromatic and olefin comonomers may be selected from the group consisting of an (AB)ₓ multiple block copolymer structure where x > 1, ABA triblock copolymer, ABA triblock in combination with AB diblock copolymer structures, (AB)ₓ multiple block copolymer where x > 1 in combination with AB diblock copolymer structures, and combinations thereof

The adhering component, which may include a film or foam, and the attachment component are composed of materials selected from the group consisting of polyethylene (PE), polypropylene (PP), poly(ethylene-vinylacetate) (EVA), poly(vinyl chloride) (PVC), polystyrene (PS), polyurethane (PU), poly(ethylene terephthalate) (PET), poly(ether amide), poly(ester amide), poly(ether ester), and their copolymers.

Reference may be made under Article 54(3) EPC to EP-A-1424088 which describes an ostomy device having a pressure sensitive adhesive comprising a polysiloxane, or a polysiloxane and silicate resin including their blends and reaction products. Other adhesives disclosed in test comparisons include the adhesives HL 2816, HL 2110 and HL 2198 from H.B Fuller Company.

Reference may also be made to WO 9603167 which describes an ostomy appliance or wound drainage device having a receptacle side component including a receptacle for receiving and storing waste solids and fluids, and a body side component for adhering to the patients skin around the stoma in which the two components are releasably sealed together through a peeled and washable based coupling mechanism. The peeled and washable based coupling mechanism is made of adhesive layers, and contains passageways which enable waste to pass through to the receptacle. terephthalate) (PET), poly(ether amide), poly(ester amide), poly(ether ester), and their copolymers.

In order to evaluate the performance of various adhesives for their ability to resist intestinal fluids, a laboratory test was developed using Simulated Ileo Fluid (SIF), a variation of the intestinal fluid test solution found in the United States Pharmacopeia. A testing jig was made from a PET sheet to which was attached an ostomy wafer of the design disclosed in Patent Application WO99/26565. Ostomy pouches of the design disclosed in Patent Application WO99/26565 were attached by an adhesive coupling onto the wafer landing zone comprised of polyethylene and poly (ethylene-co-vinyl acetate) films. This EVA film contains 9% vinyl acetate comonomer.

For each test, up to 10 pouches were then filled with 200 grams of SIF at various concentrations, sealed and inverted so that contact between their contents and the coupling interface would be continuous and intimate. Pouches were either suspended on a moving wall or laid flat. Environmental conditions were maintained at a temperature of 40C and not less than 75% relative humidity to simulate conditions of use.

After approximately 12 - 24 hours the pouches were removed from their environmental chamber and returned to the laboratory for evaluation of the resistance of the coupling to the simulated ileo fluid. Testing was conducted at various concentrations of SIF to account for individual variations in stomal output. Ratings of migration were made according to the radial distance traveled by the SIF through the coupling system. The radial distance from inner diameter to outer diameter of the coupling is approximately one inch. The following results were obtained:

**Table 1: Migration Results**

| SIF Concentration | Low | Medium | High |
|---|---|---|---|
| Typical Migration Ratings for ostomy device disclosed in WO99/26565 | 0-1 | 1-3 | 3-5 |

**Table 2: Migration Test Rating Scale**

| Rating Scale | 0 | 1 | 2 | 3 | 4 | 5 |
|---|---|---|---|---|---|---|
| Migration Distance | None | Up to 0.125 inches from coupling ID | Up to 0.25 inches from coupling ID | Up to 0.5 inches from coupling ID | Migrated to outer edge of coupling | Leaked through coupling entirely |

These typical results are similar to actual clinical results for devices of the described design, indicating a correlation between the laboratory test and device performance in actual use.

Additional pouches were produced having foam according to the design disclosed in WO99/26565, incorporated herein by reference, with the exception that the adhesive component of the pressure sensitive adhesive coupling was replaced with various other adhesives according to the following tables. These pouches were tested according to the method described above. In some cases the adhesive coupling was adhered directly to an EVA sheet without an ostomy wafer being used, the sheet having composition and dimensions equivalent to those of the wafer attachment component. In that case the EVA sheet to which the pouch was attached was then further adhered to the test jig on its side opposite from the pouch. The EVA sheet was approximately 0.010 inches thick, comprised of polyethylene with 9% vinyl acetate comonomer (polyethylene vinyl acetate), and was the primary attachment point on the body attaching component, sometimes referred to as a landing zone, a term sometimes used to describe the adhesive attachment for securing diaper tabs. The results are listed in Table 3. A migration result is shown for each individual pouch tested at the condition indicated.

**Table 3: Migration Ratings for Various Adhesives Evaluated for Ostomy Couplings (Elapsed Time 12-24 hrs)**

| Adhesive ID | Adhesive Component | Low | Medium | High |
|---|---|---|---|---|
| ¹Duro-Tak^{®} 87-4098 | Polyacrylate | 0,0,0 | 3,2,1 | 4,4,4 |
| ¹Duro-Tak^{®} 87-9085 | Polyisobutylene | 1,1,1 | 2,3,2 | 3,4,3 |
| ¹Duro-Tak^{®} 87-9301 | Polyacrylate | 2,2,2 | 3,3,3 | 3,4,4 |
| ¹Duro-Tak^{®} 87-9088 | Polyacrylate | 1,1,1 | 3,2,2 | 5,5,5 |
| ²GMS 2999 | Polyacrylate | 2,1,2 | 2,2,2 | 3,3,3 |
| ²GMS 1753 | Polyacrylate | 1,1,0 | 4,3,3 | 3,5,5 |
| ²GMS 2495 | Polyacrylate | 0,1,2 | 3,3,3 | 5,5,5 |
| ²GME 2484 | Polyacrylate | 4,5,5 | 5,5,5 | 5,5,5 |
| ²GME 3060 | Polyacrylate | 5,5,5 | 5,5,5 | 5,5,5 |
| ³HL 2816 | Styrenic Block Copolymer | NA | NA | 5 |
| ³HL-2110 | Styrenic Block Copolymer | NA | 2 | 5 |
| ³HL-2198 | Styrenic Block Copolymer | NA | 2 | 5 |
| ⁴Gel 2000 | Hydrogel | 5,5,5 | 5,5,5 | 5,5,5 |
| ⁵Mediderm 3603 | Polyisobutylene | 5 | 5 | 5 |

| | | | | |
|---|---|---|---|---|
| ¹ Duro-Tak® is a trademark of the National Starch and Chemical Company (Division of ICI). ²GMS and GME stand for Gelva® Multipolymer Solution and Gelva® Multipolymer Emulsion, trademarks of Solutia, Inc. ³H. B. Fuller Company ⁴ Conmed Corporation ⁵ Mylan Technologies, Inc | | | | |

The results in Table 3 show that all of the adhesives exhibited significant migration of SIF through the adhesive coupling at the interface between the adhesive and the EVA sheet. Migration was found to depend on the chemical composition of the adhesive itself and appeared to be independent of the level of adhesion between the coupling components.

Additional testing was conducted in order to discover adhesives that resist intestinal fluid. It was believed that adhesives comprised of polymers that exhibit good chemical resistance would also resist stomal effluent. For that reason adhesives comprised of styrenic block copolymers, and polyisobutylene were tested. Commercially available adhesives of this kind did not prevent migration of the SIF test solution. Experiments were undertaken to determine if certain adhesive compositions based on multi-block copolymers of styrene and olefin could be found that would resist stomal effluent.

### Pressure Sensitive Adhesive formulations based on Multi-block Copolymers of vinyl aromatic and olefin comonomers

Many multi-block copolymers of vinyl aromatic monomers such as styrene and olefins are known. It is not known as provided in the present invention to use multi-block copolymers of vinyl aromatic and olefin comonomers in an adhesive that forms the coupling mechanism between two components of an ostomy device to contain and resist stomal effluent. The multi-block copolymers that are effective include ABA block copolymers, referred to as "triblock" copolymers, wherein the A component is a non-elastomeric component such as styrene and the B component is an elastomeric component such as an olefin. The triblock copolymers may also contain AB block copolymers, referred to as "diblock" copolymers. In addition, the multi-block copolymer may include (AB)ₓ multiple blocks, where x > 1. For example, (AB)ₓ₌₄ = ABABABAB. The olefins that are effective include isoprene, butadiene, ethylene-propylene, ethylene-butylene, isobutylene, and ethylene-ethylene-propylene and combinations thereof. The B component of the block copolymers could be saturated and/or unsaturated. The adhesives of this invention may be formulated with tackifiers, plasticizers, antioxidants, and other additives such as fillers, pigments, etc. The tackifiers that may be used include hydrocarbon resins (aliphatic, aromatic, and combinations of the two), terpenes, terpene-phenolics, styrenated terpenes, rosins, rosin esters, etc.
Plasticizers are generally added to lower the viscosity of the adhesive solution or melt and the stiffness of the dried adhesive. These plasticizers could be mineral oils that are naphthenic or paraffinic, organic compounds such as dioctyl phthalate and dioctyl adipate, and waxes, such as polar or non-polar waxes.
Antioxidants may be added to prevent degradation of the block copolymer from heat and light. These antioxidants, depending on their chemistry, provide stability in solution and/or melt state and during the service life of the adhesive.

### Examples of Styrenic Block Copolymer adhesive compositions

See Table 4 for a description of the adhesive ingredients.

### SIS rubber adhesives

A mixing container was filled with 230 grams of heptane, placed on a heating plate, and stirred constantly with a propeller shaped mixing blade at a speed of between 50 and 100 rpm. To the stirring solvent was added 120 grams of Vector 4111 poly(styrene-isoprene-styrene) (SIS) block copolymer. The mixture was heated nearly to the boiling point and allowed to reach a temperature of approximately 90 ºC. When the polymer had fully dissolved in the solvent 80 grams of Escorez 2510, an aromatic modified aliphatic tackifying resin, was added and dissolved into the polymer solution. To this mixture was added 20 grams of Jayflex 210, a naphthenic oil for plasticizing the polymeric components of the adhesive. The solution was mixed until a homogeneous blend was obtained. Those skilled in the art will recognize that many methods of solvent blending are suitable for preparing the composition described above and that the process in no way limits the invention disclosed herein. The preferred styrenic block copolymer is the triblock polymer of styrene-isoprene-styrene, Vector 4111, as shown in the examples.

A portion of the blend was removed and drawn over siliconized paper using a coating knife to control the thickness of the adhesive layer to about 0.003 inches. Solvent from the adhesive layer was allowed to dry at ambient conditions for 5 minutes, and then to dry for 5 minutes in an oven at 105 °C. The resultant adhesive layer was substantially solvent free and was then laminated with a closed cell foam comprised of a cross linked copolymer of ethylene and vinyl acetate (EVA). The adhesive coated foam prepared in this manner was similar to that described in WO99/26565 with the noted exception of the adhesive composition. Annular sections of the adhesive coated foam were cut from the sheet and welded to panels of plastic film and converted to ostomy pouches of the kind described in WO99/26565. Those skilled in the art will recognize that there are many suitable methods for preparing the coated foam and ostomy pouches described above and that the process in no way limits the invention disclosed herein. Using the same or similar conditions other adhesive formulations were prepared using the following components and according the schedule in Table 5.

**Table 4: Description of Adhesive Ingredients**

| **Material** | **Composition** | **Available from** |
|---|---|---|
| Vector 4111 | Poly(styrene-isoprene-styrene) Block Copolymer | Dexco Polymers |
| Piccotac 1095 | Aliphatic Hydrocarbon Tackifying Resin | Eastman Chemical Company |
| Jayflex 210 | Naphthenic Process Oil | ExxonMobil Chemical Co. |
| Escorez 2510 | Aromatic Modified Aliphatic Resin | ExxonMobil Chemical Co. |
| Mineral Oil | Paraffinic Mineral Oil, USP Grade | Crompton Corporation |
| Pentalyn H | Hydrogenated Rosin Ester Tackifier | Hercules Inc. |
| Septon S2063 | Poly(styrene-ethylene-propylene-styrene) block copolymer | Septon Company of America |
| Septon S2004 | Poly(styrene-ethylene-propylene-styrene) block copolymer | Septon Company of America |
| Septon S8007 | Poly (styrene-ethylene-butylene-styrene) | Septon Company of America |
| Septon S4033 | Poly(styrene-ethylene-ethylene-propylene-styrene) | Septon Company of America |
| SIBSTAR 073T | Poly(styrene-isobutylene-styrene) block copolymer (30% styrene; mol. wt. 65,000 g/mol | Kaneka Corporation |
| SIBSTAR 103T | Poly(styrene-isobutylene-styrene) block copolymer (30% styrene; mol. wt. 100,000 g/mol) | Kaneka Corporation |
| Stereon 841A | Styrene-butadiene multiblock copolymer | Firestone Co. |
| Kraton D1102 | Poly(styrene-butadiene-styrene) block copolymer | Kraton Polymers |
| Escorez 5300 | Aliphatic hydrocarbon resin | ExxonMobil Co |
| Escorez 5600 | Aliphatic aromatic hydrocarbon resin | ExxonMobil Co |
| Sylvares TR 1135 | Terpene resin | Arizona Chemical Co. |
| Sylvares ZT 5100 | Styrenated terpene resin | Arizona Chemical Co. |
| Sylvares TR1085 | Terpene resin | Arizona Chemical Co. |
| Sylvalite RE80 LT | Hydrogenated rosin ester | Arizona Chemical Co. |
| Hercolite 240 (Plastolyn 240) | poly(alpha methyl styrene) resin | Eastman Chemical Co. |
| Kaydol oil | Paraffinic process oil | Crompton/Witco corporation |

**Table 5: SIS rubber adhesive formulations for Pouch Attachment**

| Mix | 1 | 2 | 3 | 4 | 5 | 6 |
|---|---|---|---|---|---|---|
| Vector 4111 | 54.5% | 69.8% | 69.8% | 63.2% | 69.8% | 69.8% |
| Piccotac 95 | -- | 20.9% | -- | 26.3% | 20.9% | -- |
| Jayflex 210 | 9.1% | 9.3% | 9.3% | 10.5% | -- | 9.3% |
| Escorez 2510 | 36.4% | -- | 20.9% | -- | -- | -- |
| Mineral Oil | -- | -- | -- | -- | 9.3% | -- |
| Pentalyn H | -- | -- | -- | -- | -- | 20.9% |

The EVA foam of WO99/26565 was coated with each of the formulations and tested for peel strength from the landing zone (EVA film) of ostomy wafers of the design disclosed in Patent Application WO99/26565. Pouches were prepared from these adhesive coated foams and tested using the SIF test solution. The results are listed in Table 6. A migration result is shown for each individual pouch tested at the condition indicated. Results for control pouches show actual results for the control at the condition indicated.

**Table 6: Migration Results for SIS rubber Adhesives (Elapsed Time 12 - 24 hours)**

| Mix | Control | 1 | 2 | 3 | 4 | 5 | 6 |
|---|---|---|---|---|---|---|---|
| Peel Strength* | 1.5 - 2.5** | 3.9 | 1.32 | 1.17 | 1.38 | 1.36 | 0.79 |
| (Newtons/inch) | | | | | | | |
| Migration Rating | 0, 0, 0 | | | 1, 1, 0 | | 0, 1 | 0, 0, 0 |
| SIF Concentration = Medium | | | | | | | |
| Migration Rating | 4, 4, 0 | | | 2, 2, 2 | | 2, 0, 0 | 0, 0, 0 |
| SIF Concentration = High | | | | | | | |
| Migration Rating | | | | | | | |
| SIF Concentration = Very | | | | | | | |
| High | 5, 5, 5 | | | 0, 0, 0 | | 2, 2, 0 | 0, 0, 0 |
| (Very High = 1.5 X High | | | | | | | |
| Concentration) | | | | | | | |
| Migration Rating | 4, 4, 4 | | | 5, 3, 3 | 2, 3, 0 | 5, 5 | 0, 0, 0 |
| SIF Concentration = 8 Times High | | | | | | | |

| | | | | | | | |
|---|---|---|---|---|---|---|---|
| * Per ASTM D3330 using an ostomy wafer landing zone substrate comprised of poly(ethylene co-vinyl acetate) film with 9% vinyl acetate content. ** Typical range of results for control foam. | | | | | | | |

Further testing of the foam coated with the adhesives per ASTM D3330 showed a peel strength from stainless steel of 3.0 to 21.0 Newtons/inch.

Additional testing was conducted by the same method as above except using an elapsed time of testing of approximately 240 hours. Results are shown in Table 7.

**Table 7: Long Time Migration Results for SIS-rubber Adhesives (Approximately 240 hours)**

| Mix | Control | 1 | 2 |
|---|---|---|---|
| Migration Rating | 5, 3, 4 | 0, 0, 0 | 1,1,0 |
| SIF Concentration = Low | | | |
| Migration Rating | 5, 1, 3 | 0, 0, 0 | 2, 2, 2 |
| SIF Concentration = Medium | | | |
| Migration Rating | 5, 5 | 5, 4, 2 | 2, 2, 2 |
| SIF Concentration = High | | | |

The results show significant improvement of the adhesives listed in Table 5 above compared with those listed in either table 1 or Table 3. It is believed that the use of a naphthenic oil, either alone or in combination with a tackifying agent having some compatibility with the styrene end block causes the migration resistance demonstrated by these formulations. Improvement over the control is also shown using paraffinic oil, though the improvement is not as significant. The preferred adhesive for the coupling mechanism comprises about 70% of a triblock, styrene-isoprene-styrene copolymer, blended with about 10% of a plasticing oil, most preferably a naphthenic oil, and about 20% of a hydrogenated rosin ester tackifying gum. Other suitable tackifiers include but are not limited to rosin or rosin ester based tackifiers, terpenes including alpha- and beta- pinenes (Piccolye S115) and d-limonene, aliphatic resins (Piccotac (1095), and mixed aliphatic-aromatic resins or aromatic modified aliphatic resins (Escorez 2510, Piccotac 8095).

### SEPS rubber adhesives

SEPS rubber adhesive mixtures were prepared using the mix procedure mentioned above. The formulations are shown in Table 8. The adhesive coated foam was used to prepare the pouches using the procedure outlined above and tested using the SIF test solution. The migration test results are shown in Table 9.

**Table 8: SEPS rubber adhesive formulations for Pouch Attachment**

| Mix | 7 | 8 | 9 | 10 | 11 | 12 | 13 | 14 | 15 |
|---|---|---|---|---|---|---|---|---|---|
| S2063 | 35% | -- | -- | -- | -- | -- | -- | -- | 50% |
| S2004 | 15% | 50% | 40% | 30% | 20% | 60% | 50% | 50% | -- |
| Escorez 5600 | 40% | 40% | 45% | 50% | 60% | 30% | 50% | -- | 50% |
| Sylvares TR1085 | -- | -- | -- | -- | -- | -- | -- | 40% | -- |
| Kaydol oil | 10% | 10% | 15% | 20% | 20% | 10% | -- | 10% | -- |

**Table 9: Migration Results for SEPS rubber Adhesives (Elapsed Time 12 - 24 hours)**

| Mix | Con- trol | 7 | 8 | 9 | 10 | 11 | 12 | 13 | 14 | 15 |
|---|---|---|---|---|---|---|---|---|---|---|
| Peel Strength *Newtons/ inch | 1.5- 2.5** | 2.0 | 1.4 | 2.2 | 3.7 | 1.8 | 0.3 | 0.6 | 0.7 | 4.6*** |
| Migration rating (SIF concentration= High) | 3,3, 4 | 0, 0, 0, 0 | 0,0- 1,0 | 0, 0, 0, 0 | 2,2, 1, 2 | 3-4, 5, 4, 5 | 1,2, 1,2 | 1, 0, 1, 1 | 1, 1, 1-2, 2 | 0,0,0, 1 |

| | | | | | | | | | | |
|---|---|---|---|---|---|---|---|---|---|---|
| * Per ASTM D3330 using an ostomy wafer landing zone substrate comprised of poly(ethylene co-vinyl acetate) film with 9% vinyl acetate content. ** Typical range of results for control foam ***Severe foam stretching during peel test | | | | | | | | | | |

It can be seen from Table 8 that the migration resistance of the SEPS rubber formulations is superior to the control adhesive. A preferred composition for adhesive coupling with SEPS rubber comprises of about 50% of poly(styrene-ethylene-propylene-styrene) rubber, about 40% of an aliphatic-aromatic tackifier, and about 10% of mineral oil.

### SIBS rubber adhesives

SIBS (poly(styrene-isobutylene-styrene) rubber adhesive mixtures were prepared using the mix procedure mentioned above. The formulations are shown in Table 10. The adhesive coated foam was used to prepare the pouches using the procedure outlined above and tested using the SIF test solution. The migration test results are shown in Table 11. It can be seen from Table 11 that the migration resistance of the SIBS rubber formulations is superior to the control adhesive. A preferred composition for adhesive coupling with SIBS rubber comprises of about 50% of poly(styrene -isobutylene-styrene) rubber, about 40% of an aliphatic-aromatic tackifier, and about 10% of mineral oil.

Even though the above examples describe adhesives mixed and coated using a solvent media, those skilled in the art will recognize that the adhesives can also be mixed in the melt state without the use of solvents. Likewise, the coating process can also be accomplished by various methods in solvent or melt.

The amount of tackifier and plasticizing oil can be varied across a wide range to yield useful pouch attaching adhesives. However, the peel strength does have practical limits beyond which the ostomy device will not be acceptable. The peel strength of the adhesive coated foam from the body attaching component should not be so low as

**Table 10: SIBS rubber adhesive formulations for Pouch Attachment**

| Mix | 16 | 17 | 18 |
|---|---|---|---|
| SIBSTAR 073T | 60% | -- | -- |
| SIBSTAR 103T | -- | 60% | 50% |
| Escorez 5600 | -- | -- | -- |
| Sylvares TR 1135 | 30% | 30% | 40% |
| Kaydol oil | -- | -- | 10% |
| Jayflex oil | 10% | 10% | -- |

**Table 11: Migration Results for SIBS rubber Adhesives (Elapsed Time 12 - 24 hours)**

| Mix | Control | 16 | 17 | 18 |
|---|---|---|---|---|
| Peel Strength* (Newtons/inch) | 1.5-2.5** | 0.6 | 0.4 | 0.6 |
| Migration Rating SIF Concentration = High | 3, 4, 4 | 2, 2, 2 | 1-2, 1-2, 1-2 | 0, 0, 0 |

| | | | | |
|---|---|---|---|---|
| * Per ASTM D3330 using an ostomy wafer landing zone substrate comprised of poly(ethylene co-vinyl acetate) film with 9% vinyl acetate content. ** Typical range of results for control foam | | | | |

to cause the attachment of the pouch to the wafer to be insecure or susceptible to leaks. A lower limit of acceptable peel strength for this purpose is believed to be about 0.3 Newtons/inch. Also, peel strength of the adhesive coated foam from the body attaching component should not be so high as to cause the wafer to be either loosened, or partially or completely removed from the skin by removal of the pouch from the wafer. Furthermore, the peel strength cannot be so high that the adhesive delaminates from the substrate (in this case an EVA foam) and remains on the body attaching component. If delamination occurs then the residue prevents subsequent pouches from achieving a perfect seal to the adulterated attachment point, and that would defeat the reusability of the body attaching component. An upper limit of acceptable peel strength for this purpose is believed to be about 6.0 Newtons/inch. Formulations of styrenic block copolymer, tackifier and plasticizing oil with peel strength from landing zone material between about 0.3 and about 6.0 Newtons/inch result in useful pouch attaching adhesives that resist attack by simulated ileo fluid (SIF), and are believed to be resistant to stomal effluent. These formulations have also been found to have a peel strength from stainless steel per ASTM D3330 of about 3.0 to about 21.0 Newtons/inch, preferably from about 5.0 to about 18.0 Newtons per inch.

### SEBS, SBS, and SEEPS rubber examples

Further more, Table 12 shows the formulations of SEBS, SBS, and SEEPS rubber-based adhesives. As it can be seen, these compositins are resistant to simulated ileal fluid for at least 12 hrs., and are suitable as ostomy pouch attachment adhesives.

**Table 12: SEBS, SBS, and SEEPS rubber adhesive formulations for Pouch Attachment**

| Mix | 19 | 20 | 21 | 22 |
|---|---|---|---|---|
| S8007 | 50% | -- | -- | -- |
| S4033 | -- | 50% | -- | -- |
| Stereon 841A | -- | -- | 60 | -- |
| Kraton D1102 | -- | -- | -- | 50 |
| Escorez 5600 | 40% | 40% | 30 | 40 |
| Kaydol oil | 10% | 10% | 10 | 10 |
| Peel Strength* (Newtons/inch) | 0.6 | 0.6 | 1.3 | 2.1 |
| Migration Rating SIF concentration = High (Elapsed Time 12-24hrs) | 2-3, 2-3, 2 | 2,5**, 5** | 1-2, 2, 1-2 | 0-1, 1-2, 0-1 |

| | | | | |
|---|---|---|---|---|
| * Per ASTM D3330 using an ostomy wafer landing zone substrate comprised of poly(ethylene co-vinyl acetate) film with 9% vinyl acetate content. **Migration of SIF due to improper welding of foam collar to pouch | | | | |

### EVA based adhesives

Additional experiments were conducted using Duro-Tak H1509, a hot melt poly(ethylene vinyl acetate) adhesive with pressure sensitive properties. Migration test results are shown in Table 13. These results indicate that migration resistance similar to that obtained for styrenic block copolymers can be obtained using other rubbery polymers suitable for pressure sensitive adhesives, for example poly(ethylene vinyl acetate). Adhesives based on EVA polymers are known in the prior art. Preferred compositions are those comprising between about 10 and 50% vinyl acetate content, more preferably about 30 to about 45%. Suitable tackifiers are similar to those described above and preferably include rosin esters, terpenes, terpene phenolics, hydrocarbon resins (aromatic, aliphatic, and combinations of the two), and combinations thereof. It is known that formulations with EVA polymers can also contain a wax or mineral oil as a process aid or plasticizer. The waxes could be petroleum or mineral waxes. In addition, there are synthetic waxes that can also be incorporated.

**Table 13: Average Migration Results for EVA Pressure Sensitive Adhesive**

| Adhesive ID | Adhesive Component | Low | Medium | High |
|---|---|---|---|---|
| ¹Duro-Tak^{®} H1509 | Poly(ethylene-co- vinyl acetate) | 0.4 | 1.2 | 2.4 |

| | | | | |
|---|---|---|---|---|
| ¹ Duro-Tak^{®} is a trademark of the National Starch and Chemical Company (Division of ICI). | | | | |

## Claims

1. An ostomy device comprising an attachment component for attachment to the body, an effluent containment component secured to the attachment component, and a coupling mechanism for securing the effluent containment component to the attachment component, wherein:
the coupling mechanism includes an adhering component with an adhesive that comprises either a multi-block copolymer of vinyl aromatic and olefin comonomers, or poly(ethylene vinyl acetate), or combinations thereof,
the adhesive resists migration of effluent for at least 12 hours; and
when the adhesive comprises a multi-block copolymer of vinyl aromatic and olefin comonomers:
said multi-block copolymer comprises from about 20 to about 85 percent by weight of the dry adhesive, the adhesive further includes a plasticizer comprising from about 0 to about 40 percent by weight of the dry adhesive, and the adhesive further includes a tackifier comprising from about 5 to about 60 percent by weight of the dry adhesive.

2. The ostomy device of claim 1 wherein the device is a one-piece or a two-piece appliance.

3. The ostomy device of claim 1 wherein the device is a two-piece appliance, and the adhesive permits repositioning of one piece relative to the other.

4. The ostomy device of claim 1 wherein the multi-block copolymer of vinyl aromatic and olefin comonomers is selected from a group consisting of poly (styrene-isoprene-styrene), poly (styrene-butadiene-styrene), poly (styrene ethylene-butylene-styrene), poly (styrene-ethylene-propylene-styrene), poly (styrene-isobutylene-styrene), poly (styrene-ethylene-ethylene-propylene-styrene), and combinations thereof.

5. The ostomy device of claim 1 wherein the plasticizer is an oil.

6. The ostomy device of claim 1 wherein the plasticizer is a wax.

7. The ostomy device of claim 1 wherein the plasticizer is an adipate or a phthalate.

8. The ostomy device of claim 1 wherein the multi-block copolymer of vinyl aromatic and olefin comonomers includes an ABA triblock copolymer structure.

9. The ostomy device of claim 1 wherein the multi-block copolymer of vinyl aromatic and olefin comonomers includes a combination of ABA triblock and AB diblock copolymer structures.

10. The ostomy device of claim 1 wherein the multi-block copolymer of vinyl aromatic and olefin comonomers includes an (AB)ₓ multiple block copolymer structure, where x > 1.

11. The ostomy device of claim 1 wherein the multi-block copolymer of vinyl aromatic and olefin comonomers is selected from the group consisting of an (AB)ₓ multiple block copolymer structure where x > 1, ABA triblock copolymer, ABA triblock in combination with AB diblock copolymer structures, (AB)ₓ multiple block copolymer where x > 1 in combination with AB diblock copolymer structures, and combinations thereof.

12. The ostomy device of claim 5 wherein the plasticizing oil is either a naphthenic oil or a paraffinic mineral oil.

13. The ostomy device of claim 1 wherein the tackifier is selected from the group consisting of rosins, rosin esters, terpenes, terpene-phenolics, aliphatic resins, mixed aliphatic-aromatic resins, aromatic-modified aliphatic resins, aromatic resins, and combinations thereof.

14. The ostomy device of claim 1 wherein the poly(ethylene vinyl acetate) has a vinyl acetate content between about 10% and about 50% and is blended with a tackifier selected from the group consisting of rosins, rosin esters, terpenes, terpene-phenolics, aliphatic resins, mixed aliphatic-aromatic resins, aromatic-modified aliphatic resins, aromatic resins, and combinations thereof.

15. The ostomy device of claim 14 wherein the poly(ethylene vinyl acetate) and tackifier are also blended with a plasticizing oil.

16. The ostomy device of claim 14 wherein the poly(ethylene vinyl acetate) and tackifier are also blended with a wax.

17. The ostomy device of claim 1 wherein multi-block copolymer of vinyl aromatic and olefin comonomers comprises from about 20 to about 85 percent by weight of the dry adhesive.

18. The ostomy device of claim 1 wherein the adhering component includes a film or a foam.

19. The ostomy device of claim 18 wherein the film or foam is selected from the group consisting of polyethylene (PE), polypropylene (PP), poly(ethylenevinylacetate) (EVA), poly(vinyl chloride) (PVC), polystyrene (PS), polyurethane (PU), poly(ethylene terephthalate) (PET), poly(ether amide), poly (ester amide), poly (ether ester), and their copolymers.

20. The ostomy device of claim 1 wherein the attachment component includes materials selected from the group consisting of polyethylene (PE), polypropylene (PP), poly(ethylene-vinyl acetate) (EVA), poly(vinyl chloride) (PVC), polystyrene (PS), polyurethane (PU), poly(ethylene terephthalate) (PET), poly(ether amide), poly(ester amide), poly (ether ester), and their copolymers.

21. The ostomy device of claim 18 wherein the adhering component is a foam coated with an adhesive, and the foam coated with an adhesive has a peel strength from stainless steel per ASTM D3330 from about 3.0 to about 21.0 Newtons/inch.

22. The ostomy device of claim 18 wherein the adhering component is a foam coated with an adhesive, and the foam coated with an adhesive has a peel strength from the attachment component from about 0.3 to about 6.0 Newtons/inch using the test method of ASTM D3330 wherein the stainless steel substrate is replaced with the attachment component material.

23. The ostomy device of claim 1 wherein the device is a one-piece appliance having the attachment component and effluent containment component adhesively secured together.

## Patentansprüche

1. Ostomiehilfsmittel mit einer Halterungskomponente für die Halterung am Körper, einer Ausflussrückhaltekomponente, die an der Halterungskomponente befestigt ist, und einem Kopplungsmechanismus für das Befestigen der Ausflussrückhaltekomponente an der Halterungskomponente, wobei:
der Kupplungsmechanismus eine Haftkomponente mit einem Klebemittel enthält, das entweder ein Mehrblock-Copolymer aus vinylaromatischen und Olefin-Comonomeren oder Poly(ethylenvinylacetat) oder Kombinationen davon enthält,
das Klebemittel der Ausbreitung des Ausflusses für mindestens 12 Stunden widersteht, und dass dann,
wenn das Klebemittel ein Mehrblock-Copolymer aus vinylaromatischen und Olefin-Comonomeren enthält:
das Mehrblock-Copolymer von etwa 20 bis zu etwa 85 Gewichtsprozent des trockenen Klebemittels umfasst, das Klebemittel ferner einen Weichmacher enthält, der von etwa 0 bis etwa 40 Gewichtsprozent des trockenen Klebemittels umfasst, und das Klebemittel ferner einen Klebrigmacher enthält, der von etwa 5 bis etwa 60 Gewichtsprozent des trockenen Klebemittels umfasst.

2. Ostomiehilfsmittel nach Anspruch 1, wobei das Hilfsmittel eine einteilige oder eine zweiteilige Vorrichtung ist.

3. Ostomiehilfsmittel nach Anspruch 1, wobei das Hilfsmittel eine zweiteilige Vorrichtung ist und das Klebemittel eine Neupositionierung des einen Teils bezüglich des anderen erlaubt.

4. Ostomiehilfsmittel nach Anspruch 1, wobei das Mehrblock-Copolymer aus vinylaromatischen und Olefin-Comonomeren aus einer Gruppe ausgewählt ist, die aus Poly(styrol-isopren-styrol), Poly(styrol-butadien-styrol), Poly(styrol-ethylen-butylen-styrol), Poly(styrol-ethylen-propylen-styrol), Poly(styrol-isobutylen-styrol), Poly(styrol-ethylen-ethylen-propylen-styrol) und Kombinationen davon besteht.

5. Ostomiehilfsmittel nach Anspruch 1, wobei der Weichmacher ein Öl ist.

6. Ostomiehilfsmittel nach Anspruch 1, wobei der Weichmacher ein Wachs ist.

7. Ostomiehilfsmittel nach Anspruch 1, wobei der Weichmacher ein Adipat oder ein Phthalat ist.

8. Ostomiehilfsmittel nach Anspruch 1, wobei das Mehrblock-Copolymer aus vinylaromatischen und Olefin-Comonomeren eine ABA-Triblock-Copolymerstruktur enthält.

9. Ostomiehilfsmittel nach Anspruch 1, wobei das Mehrblock-Copolymer aus vinylaromatischen und Olefin-Comonomeren eine Kombination aus ABA-Triblock- und AB-Diblock-Copolymerstrukturen enthält.

10. Ostomiehilfsmittel nach Anspruch 1, wobei das Mehrblock-Copolymer aus vinylaromatischen und Olefin-Comonomeren eine (AB)ₓ-Mehrblock-Copolymerstruktur mit x > 1 enthält.

11. Ostomiehilfsmittel nach Anspruch 1, wobei das Mehrblock-Copolymer aus vinylaromatischen und Olefin-Comonomeren aus der Gruppe ausgewählt ist, die aus einer (AB)ₓ-Mehrblock-Copolymerstruktur mit x > 1, einem ABA-Triblock-Copolymer, ABA-Triblock- in Kombination mit AB-Diblock-Copolymerstrukturen, einem (AB)ₓ-Mehrblock-Copolymer- mit x > 1 in Kombination mit AB-Diblock-Copolymerstrukturen und Kombinationen davon besteht.

12. Ostomiehilfsmittel nach Anspruch 5, wobei das weichmachende Öl entweder ein naphthenisches Öl oder ein paraffinisches Mineralöl ist.

13. Ostomiehilfsmittel nach Anspruch 1, wobei der Klebrigmacher aus der Gruppe ausgewählt ist, die aus Naturharzen, Naturharzestern, Terpenen, Terpen-Phenolen, aliphatischen Harzen, gemischten aliphatisch-aromatischen Harzen, aromatisch-modifizierten aliphatischen Harzen, aromatischen Harzen und Kombinationen davon besteht.

14. Ostomiehilfsmittel nach Anspruch 1, wobei das Poly(ethylenvinylacetat) einen Vinylacetatgehalt zwischen etwa 10 % und etwa 50 % aufweist und gemischt ist mit einem Klebrigmacher, der aus der Gruppe ausgewählt ist, die aus Naturharzen, Naturharzestern, Terpenen, Terpen-Phenolen, aliphatischen Harzen, gemischten aliphatisch-aromatischen Harzen, aromatisch-modifizierten aliphatischen Harzen, aromatischen Harzen und Kombinationen davon besteht.

15. Ostomiehilfsmittel nach Anspruch 14, wobei das Poly(ethylenvinylacetat) und der Klebrigmacher auch mit einem weichmachenden Öl vermischt sind.

16. Ostomiehilfsmittel nach Anspruch 14, wobei das Poly(ethylenvinylacetat) und der Klebrigmacher auch mit einem Wachs vermischt sind.

17. Ostomiehilfsmittel nach Anspruch 1, wobei das Mehrblock-Copolymer aus vinylaromatischen und Olefin-Comonomeren von etwa 20 bis zu etwa 85 Gewichtsprozent des trockenen Klebemittels beinhaltet.

18. Ostomiehilfsmittel nach Anspruch 1, wobei die Haftkomponente einen Film oder einen Schaumstoff enthält.

19. Ostomiehilfsmittel nach Anspruch 18, wobei der Film oder Schaumstoff aus der Gruppe ausgewählt ist, die aus Polyethylen (PE), Polypropylen (PP), Poly(ethylenvinylacetat) (EVA), Poly(vinylchlorid) (PVC), Polystyrol (PS), Polyurethan (PU), Poly(ethylenterephthalat) (PET), Poly(etheramid), Poly(esteramid), Poly(etherester) und deren Copolymeren besteht.

20. Ostomiehilfsmittel nach Anspruch 1, wobei die Halterungskomponente Materialien enthält, die aus der Gruppe ausgewählt sind, die aus Polyethylen (PE), Polypropylen (PP), Poly(ethylen-vinylacetat) (EVA), Poly(vinylchlorid) (PVC), Polystyrol (PS), Polyurethan (PU), Poly(ethylenterephthalat) (PET), Poly(etheramid), Poly(esteramid), Poly(etherester) und deren Copolymeren besteht.

21. Ostomiehilfsmittel nach Anspruch 18, wobei die Haftkomponente ein Schaumstoff ist, der mit einem Klebemittel beschichtet ist, und der mit einem Klebemittel beschichtete Schaumstoff nach ASTM D3330 eine Ablösefestigkeit von rostfreiem Stahl von etwa 3,0 bis zu etwa 21,0 Newton/Zoll aufweist.

22. Ostomiehilfsmittel nach Anspruch 18, wobei die Haftkomponente ein Schaumstoff ist, der mit einem Klebemittel beschichtet ist, und der mit einem Klebemittel beschichtete Schaumstoff eine Ablösefestigkeit von der Halterungskomponente von etwa 0,3 bis zu etwa 6,0 Newton/Zoll aufweist, wobei das Testverfahren nach ASTM D3330 verwendet wird, in dem das rostfreie Stahlsubstrat durch das Material der Halterungskomponente ersetzt ist.

23. Ostomiehilfsmittel nach Anspruch 1, wobei das Hilfsmittel eine einteilige Vorrichtung ist, in der die Halterungskomponente und die Ausflussrückhaltekomponente durch Kleben aneinander befestigt sind.

## Revendications

1. Dispositif de stomie comprenant un composant de fixation destiné à la fixation au corps, un composant de retenue d'effluent fixé au composant de fixation, et un mécanisme de liaison destiné à fixer le composant de retenue d'effluent au composant de fixation, dans lequel :
le mécanisme de liaison comprend un composant d'adhérence comportant un adhésif qui comprend soit un copolymère à multi-bloc de comonomères de vinyle aromatique et d'oléfine, soit du poly(éthylène acétate de vinyle), soit des combinaisons de ceux-ci,
l'adhésif résiste à la migration d'effluent pendant au moins 12 heures, et
lorsque l'adhésif comprend un copolymère à multi-bloc de comonomères de vinyle aromatique et d'oléfine:
ledit copolymère à multi-bloc comprend d'environ 20 à environ 85 pour cent en poids de l'adhésif sec, l'adhésif comprend en outre un plastifiant comprenant d'environ 0 à environ 40 pour cent en poids de l'adhésif sec, et l'adhésif comprend en outre un agent de collage comprenant d'environ 5 à environ 60 % en poids de l'adhésif sec.

2. Dispositif de stomie selon la revendication 1, dans lequel le dispositif est un équipement à une pièce ou à deux pièces.

3. Dispositif de stomie selon la revendication 1, dans lequel le dispositif est un équipement à deux pièces et l'adhésif permet le repositionnement de la première pièce par rapport à l'autre.

4. Dispositif de stomie selon la revendication 1, dans lequel le copolymère à multi-bloc de comonomères de vinyle aromatique et d'oléfine est sélectionné à partir d'un groupe constitué du poly(styrène-isoprène-styrène), du poly(styrène-butadiène-styrène), du poly(styrène éthylène-butylène-styrène), du poly(styrène-éthylène-propylène-styrène), du poly(styrène-isobutylène-styrène), du poly(styrène-éthylène-éthylène-propylène-styrène) et des combinaisons de ceux-ci.

5. Dispositif de stomie selon la revendication 1, dans lequel le plastifiant est une huile.

6. Dispositif de stomie selon la revendication 1, dans lequel le plastifiant est une cire.

7. Dispositif de stomie selon la revendication 1, dans lequel le plastifiant est un adipate ou un phtalate.

8. Dispositif de stomie selon la revendication 1, dans lequel le copolymère à multi-bloc de comonomères de vinyle aromatique et d'oléfine comprend une structure de copolymère à trois blocs ABA.

9. Dispositif de stomie selon la revendication 1, dans lequel le copolymère à multi-bloc de comonomères de vinyle aromatique et d'oléfine comprend une combinaison de structures de copolymères à trois blocs ABA et à deux blocs AB.

10. Dispositif de stomie selon la revendication 1, dans lequel le copolymère à multi-bloc de comonomères de vinyle aromatique et d'oléfine comprend une structure de copolymère à bloc multiple (AB)ₓ, où x > 1.

11. Dispositif de stomie selon la revendication 1, dans lequel le copolymère à multi-bloc de comonomères de vinyle aromatique et d'oléfine est sélectionné à partir du groupe constitué d'une structure de copolymère à bloc multiple (AB)ₓ ABA où x > 1, un copolymère à trois blocs ABA, des structures de copolymère à trois blocs ABA en combinaison avec un copolymère à deux blocs AB, des structures de copolymère à bloc multiple (AB)ₓ où x > 1 en combinaison avec un copolymère à deux blocs AB et des combinaisons de ceux-ci.

12. Dispositif de stomie selon la revendication 5, dans lequel l'huile de plastification est soit une huile naphténique, soit une huile minérale paraffinique.

13. Dispositif de stomie selon la revendication 1, dans lequel l'agent de collage est sélectionné à partir du groupe constitué de colophanes, d'esters de colophanes, de terpènes, de terpènes-phénoliques, de résines aliphatiques, de résines mélangées aliphatiques-aromatiques, de résines aromatiques modifiées en aliphatiques, de résines aromatiques et des combinaisons de ceux-ci.

14. Dispositif de stomie selon la revendication 1, dans lequel le poly(éthylène acétate de vinyle) présente une teneur en acétate de vinyle comprise entre environ 10 % et environ 50 % et est mélangé avec un agent de collage sélectionné à partir du groupe constitué de colophanes, d'esters de colophanes, de terpènes, de terpènes-phénoliques, de résines aliphatiques, de résines mélangées aliphatiques-aromatiques, de résines aromatiques modifiées en aliphatiques, de résines aromatiques et de combinaisons de ceux-ci.

15. Dispositif de stomie selon la revendication 14, dans lequel le poly(éthylène acétate de vinyle) et l'agent de collage sont également mélangés avec une huile de plastification.

16. Dispositif de stomie selon la revendication 14, dans lequel le poly (éthylène acétate de vinyle) et l'agent de collage sont également mélangés à une cire.

17. Dispositif de stomie selon la revendication 1, dans lequel le copolymère à multi-bloc de comonomères de vinyle aromatique et d'oléfine comprend d'environ 20 à environ 85 pour cent en poids de l'adhésif sec.

18. Dispositif de stomie selon la revendication 1, dans lequel le composant d'adhérence comprend un film ou une mousse.

19. Dispositif de stomie selon la revendication 18, dans lequel le film ou la mousse est sélectionné à partir du groupe constitué du polyéthylène (PE), du polypropylène (PP), du poly(éthylène-acétate de vinyle) (EVA), du poly(chlorure de vinyle) (PVC), du polystyrène (PS), du polyuréthane (PU), du poly(téréphtalate d'éthylène) (PET), du poly(éther amide), du poly(ester amide), du poly (éther ester) et leurs copolymères.

20. Dispositif de stomie selon la revendication 1, dans lequel le composant de fixation comprend des matériaux sélectionnés à partir du groupe constitué du polyéthylène (PE), du polypropylène (PP), du poly(éthylène-acétate de vinyle) (EVA), du poly(chlorure de vinyle) (PVC), du polystyrène (PS), du polyuréthane (PU), du poly(téréphtalate d'éthylène) (PET), du poly(éther amide), du poly(ester amide), du poly (éther ester) et leurs copolymères.

21. Dispositif de stomie selon la revendication 18, dans lequel le composant d'adhérence est une mousse revêtue par un adhésif, et la mousse revêtue par un adhésif présente une résistance au décollement par rapport à l'acier inoxydable selon la norme ASTM D3330 d'environ 3,0 à environ 21,0 Newton/pouce.

22. Dispositif de stomie selon la revendication 18, dans lequel le composant d'adhérence est une mousse revêtue par un adhésif, et la mousse revêtue par un adhésif présente une résistance au décollement par rapport au composant de fixation d'environ 0,3 à environ 6,0 Newton/pouce en utilisant le procédé de test de la norme ASTM D3330 dans lequel le substrat d'acier inoxydable est remplacé par le matériau de composant de fixation.

23. Dispositif de stomie selon la revendication 1, dans lequel le dispositif est un équipement à une pièce présentant le composant de fixation et le composant de retenue d'effluent fixés ensemble par adhérence.
